(19) Europäisches Patentamt
European Patent Office
Office européen des brevets

(11) Veröffentlichungsnummer: **0 334 137 A2**

(12) **EUROPÄISCHE PATENTANMELDUNG**

(21) Anmeldenummer: 89104341.6

(22) Anmeldetag: 11.03.89

(51) Int. Cl.⁴: C07D 239/38, C07D 405/12, C07D 213/70, C07D 317/18, C07D 319/06, A01N 43/54, A01N 43/40

(30) Priorität: 24.03.88 DE 3809933

(43) Veröffentlichungstag der Anmeldung: 27.09.89 Patentblatt 89/39

(84) Benannte Vertragsstaaten: BE CH DE FR GB IT LI NL

(71) Anmelder: BAYER AG

D-5090 Leverkusen 1 Bayerwerk(DE)

(72) Erfinder: Sasse, Klaus, Dr.
Pützweg 13
D-5060 Bergisch-Gladbach 2(DE)
Erfinder: Fischer, Reiner, Dr.
Nelly-Sachs-Strasse 23
D-4019 Monheim 2(DE)
Erfinder: Hagemann, Hermann, Dr.
Kandinskystrasse 52
D-5090 Leverkusen 1(DE)
Erfinder: Krebs, Andreas, Dr.
Am Gartenfeld 70
D-5068 Odenthal-Holz(DE)
Erfinder: Santel, Hans-Joachim, Dr.
Grünstrasse 9 a
D-5090 Leverkusen 1(DE)
Erfinder: Schmidt, Robert R., Dr.
Im Waldwinkel 110
D-5060 Bergisch-Gladbach 2(DE)
Erfinder: Strang, Harry, Dr.
Unterdorfstrasse 6 a
D-4000 Düsseldorf 31(DE)

# (54) 2-Pyri(mi)dylthio-aryläther.

(57) Die Erfindung betrifft neue 2-Pyri(mi)dyl-thioarylether der Formel (I)

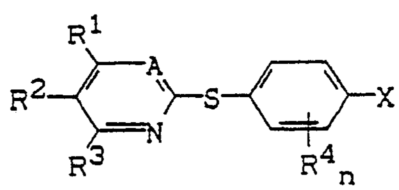

(I)

in welcher
X für die Gruppen

EP 0 334 137 A2

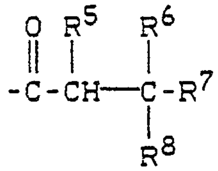 , 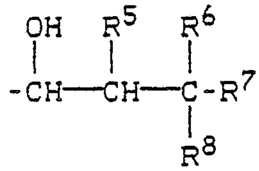 , 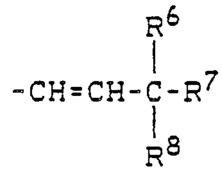 ,

(Ia) (Ib) (Ic)

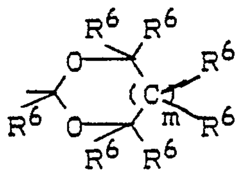 oder 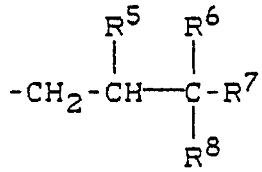

(Id) (Ie)

steht und die übrigen Substituenten die in der Beschreibung angegebene Bedeutung haben,
mehrere Verfahren sowie neue Zwischenprodukte zu deren Herstellung und deren Verwendung als Herbizide.

# 2-Pyri(mi)dylthio-arylether

Die vorliegende Erfindung betrifft neue 2-Pyri(mi)dylthioarylether, mehrere Verfahren sowie neue Zwischenprodukte zu ihrer Herstellung und ihre Verwendung als Herbizide.

Es ist bereits bekannt, daß bestimmte 2-Phenoxypyrimidine herbizide Eigenschaften besitzen (vgl. EP-A-1187 und DE-OS 2 820 032). Die Wirkung dieser Verbindungen ist gut, jedoch werden bei geringen Aufwandmengen einige Unkräuter nicht immer voll erfaßt, auch ist die Selektivität nicht immer ausreichend.

Ferner ist bekannt, daß zahlreiche Pyrimidyl-2-ether und -thioether als Herbizide geeignet sind (vgl. z.B. JP 9474/1967 (Anmeldenummer 12771/65), US-PS 3 126 271 und US-PS 3 250 775). Die herbizide Wirksamkeit dieser vorbekannten Verbindungen gegenüber Schadpflanzen ist jedoch ebenso wie ihre Verträglichkeit gegenüber wichtigen Kulturpflanzen nicht immer in allen Anwendungsgebieten zufriedenstellend.

Es wurden nun neue 2-Pyri(mi)dyl-thioarylether der Formel (I) gefunden,

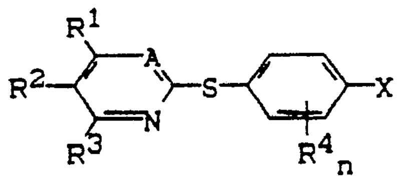

(I)

in welcher

$R^1$, $R^2$ und $R^3$ unabhängig voneinander jeweils für Wasserstoff oder Alkyl stehen,

A für eine CH-Gruppe oder ein Stickstoffatom steht,

$R^4$ für Halogen, Alkyl oder Alkoxy steht, wobei $R^4$ gleich oder verschieden sein kann,

n für eine Zahl 0, 1 oder 2 steht und

X für die Gruppen

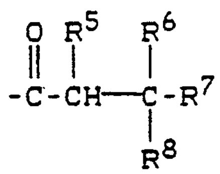 , 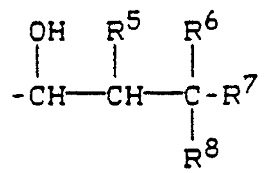 , 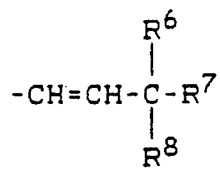 ,

(Ia) (Ib) (Ic)

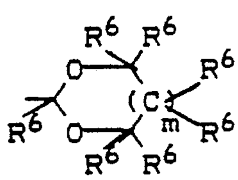 oder 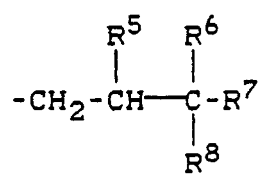

(Id) (Ie)

steht, wobei

$R^5$ für Wasserstoff, Alkyl, Halogenalkyl oder Alkoxy steht und bei der Gruppe (Ie) auch für Hydroxyl steht,

$R^6$ und $R^7$ unabhängig voneinander jeweils für Wasserstoff oder Alkyl stehen und wobei $R^6$ in der Gruppe (Id) jeweils gleich oder verschieden sein kann,

$R^8$ für Alkyl, Halogenalkyl oder Alkoxy steht oder

$R^7$ und $R^8$ gemeinsam mit dem Kohlenstoffatom, an das sie gebunden sind, für einen fünf- oder sechsgliedrigen carbocyclischen oder heterocyclischen Ring stehen und

m für die Zahl 0 oder 1 steht.

Weiterhin wurde gefunden, daß man 2-Pyri(mi)dyl-thioarylether der Formel (I) erhält, wenn man

(A) Thiophenol-Derivate der Formel (II)

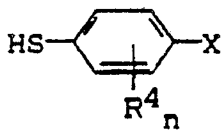

(II)

in welcher
$R^4$, X und n die oben angegebene Bedeutung haben,
mit Pyri(mi)dyl-Derivaten der Formel (III)

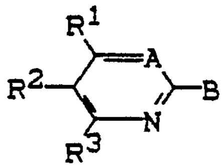

(III)

in welcher
$R^1$, $R^2$, $R^3$ und A die oben angegebene Bedeutung haben und
B für Halogen, Alkylsulfonyl oder gegebenenfalls substituiertes Arylsulfonyl steht,
in Gegenwart eines Verdünnungsmittels und gegebenenfalls in Gegenwart eines Säurebindemittels umsetzt,
oder wenn man

B) 4-Acyl-[2-pyri(mi)dyl]-thioarylether der Formel (Ia)

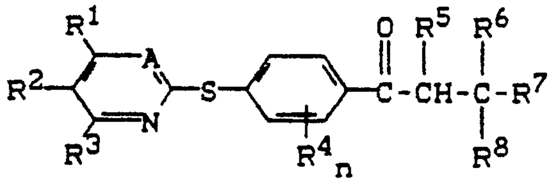

(Ia)

in welcher
$R^1$, $R^2$, $R^3$, $R^4$, $R^5$, $R^6$, $R^7$, $R^8$, A und n die oben angegebene Bedeutung haben,
zu Verbindungen der Formel (Ib)

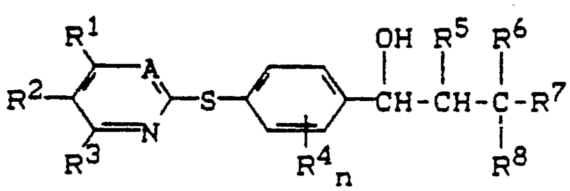

(Ib)

in welcher
$R^1$, $R^2$, $R^3$, $R^4$, $R^5$, $R^6$, $R^7$, $R^8$, A und n die oben angegebene Bedeutung haben,
in Gegenwart eines Verdünnungsmittels reduziert,
oder wenn man

C) Verbindungen der Formel (Ib)

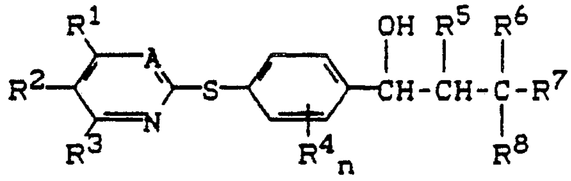

(Ib)

in welcher
$R^1$, $R^2$, $R^3$, $R^4$, $R^5$, $R^6$, $R^7$, $R^8$, A und n die oben angegebene Bedeutung haben, zu Verbindungen der Formel (Ic)

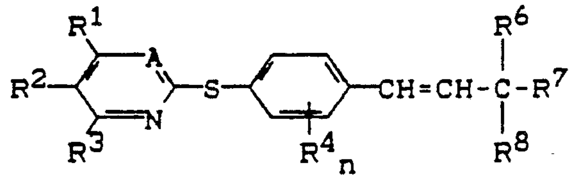

(Ic)

in welcher
$R^1$, $R^2$, $R^3$, $R^4$, $R^5$, $R^6$, $R^7$, $R^8$, A und n die oben angegebene Bedeutung haben, in Gegenwart eines Halogenierungsmittels, gegebenenfalls in Gegenwart eines Verdünnungsmittels und gegebenenfalls in Gegenwart eines Säurebindemittels umsetzt,
oder wenn man

D) Verbindungen der Formel (IV)

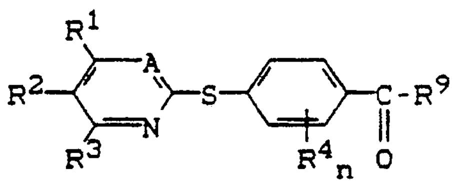

(IV)

in welcher
$R^1$, $R^2$, $R^3$, $R^4$, A und n die oben angegebene Bedeutung haben und
$R^9$ für Wasserstoff oder Alkyl steht,
mit Diolen der Formel (V)

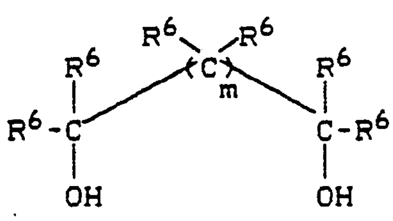

(V)

in welcher
$R^6$ und m die oben angegebene Bedeutung haben,
zu den Verbindungen der Formel (Id)

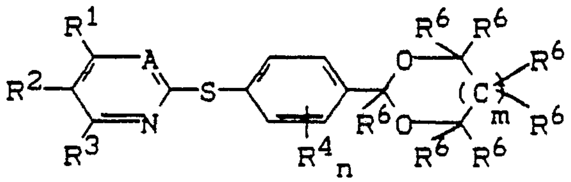

(Id)

in welcher
$R^1$, $R^2$, $R^3$, $R^4$, $R^6$, A, m und n die oben angegebene Bedeutung haben,
in Gegenwart eines Verdünnungsmittels und in Gegenwart eines Katalysators umsetzt.

Schließlich wurde gefunden, daß die neuen 2-Pyri(mi)dylthioarylether der Formel (I) herbizide Eigenschaften besitzen.

Überraschenderweise zeigen die erfindungsgemäßen 2-Pyri(mi)dyl-thioarylether der Formel (I) neben einer erheblich besseren herbiziden Wirkung gegen Unkräuter gleichzeitig eine deutlich verbesserte Nutzpflanzen-Verträglichkeit als aus dem Stand der Technik bekannte, chemisch und wirkungsmäßig naheliegende Verbindungen.

Die Kohlenstoffketten in den einzelnen Resten, wie beispielsweise Alkyl, Alkoxy, Alkylthio, Halogenalkyl oder Alkylsulfonyl, sind jeweils geradkettig oder verzweigt.

Die erfindungsgemäßen 2-Pyri(mi)dyl-thioarylether sind durch die Formel (I) allgemein definiert. Bevorzugt sind Verbindungen der Formel (I), bei welchen
$R^1$, $R^2$ und $R^3$ unabhängig voneinander jeweils für Wasserstoff oder $C_1$-$C_4$-Alkyl stehen,
A für eine CH-Gruppe oder ein Stickstoffatom steht,
$R^4$ für Halogen, $C_1$-$C_4$-Alkyl oder $C_1$-$C_4$-Alkoxy steht, wobei $R^4$ gleich oder verschieden sein kann,
n für eine Zahl 0, 1 oder 2 steht und
X für die Gruppen

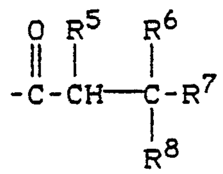 ,

(Ia)

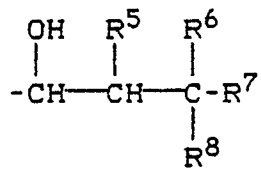 ,

(Ib)

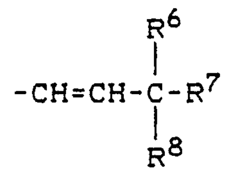 ,

(Ic)

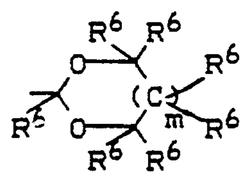

(Id)

oder

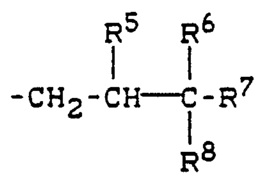

(Ie)

steht, wobei
$R^5$ für Wasserstoff, $C_1$-$C_6$-Alkyl, Halogen-$C_1$-$C_6$-alkyl oder $C_1$-$C_6$-Alkoxy und in der Gruppe (Ie) zusätzlich für Hydroxyl steht,
$R^6$ und $R^7$ unabhängig voneinander jeweils für Wasserstoff oder $C_1$-$C_6$-Alkyl stehen und wobei $R^6$ in der Gruppe (Id) jeweils gleich oder verschieden sein kann,
$R^8$ für $C_1$-$C_6$-Alkyl, Halogen-$C_1$-$C_6$-alkyl oder $C_1$-$C_6$-Alkoxy steht oder
$R^7$ und $R^8$ gemeinsam mit dem Kohlenstoffatom, an das sie gebunden sind, einen gesättigten oder ungesättigten 5- oder 6-gliedrigen Ring bilden, der außer Kohlenstoff als gleiche oder verschiedene Heteroatome 1 oder 2 Sauerstoffatome oder Schwefelatome enthalten kann, wie beispielsweise Cyclohexyl, Cyclopentyl und Tetrahydrofuranyl,

m für die Zahl 0 oder 1 steht.

Ganz besonders bevorzugt sind diejenigen Verbindungen der Formel (I), in denen $R^1$, $R^2$ und $R^3$ unabhängig voneinander jeweils für Wasserstoff, Methyl oder Ethyl stehen, A für eine CH-Gruppe oder ein Stickstoffatom steht, $R^4$ für Fluor, Chlor, Methyl, Ethyl, Methoxy oder Ethoxy steht, n für eine Zahl 0 oder 1 steht, X für die Gruppen

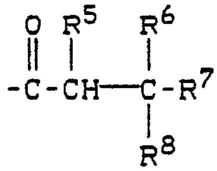 ,

(Ia)

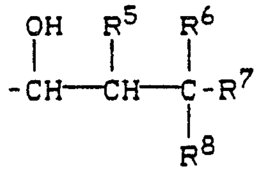 ,

(Ib)

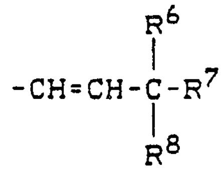 ,

(Ic)

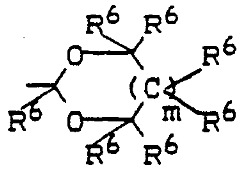

(Id)

oder

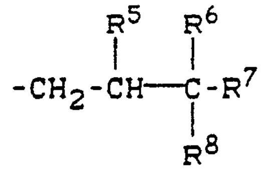

(Ie)

steht, wobei $R^5$ für Wasserstoff, Methyl, Ethyl, n-Propyl, iso-Propyl, Chlormethyl, Chlorethyl, Chlorpropyl, Methoxy, Ethoxy, n-Propoxy oder Isopropoxy und bei der Gruppe (Ie) zusätzlich für Hydroxyl steht, $R^6$ und $R^7$ unabhängig voneinander jeweils für Wasserstoff, Methyl, Ethyl, n-Propyl oder iso-Propyl stehen, wobei $R^6$ in der Gruppe (Id) gleich oder verschieden sein kann, $R^8$ für Methyl, Ethyl, n-Propyl, iso-Propyl, n-, iso-, sec- oder tert.-Butyl, Chlormethyl, Chlorethyl, Methoxy, Ethoxy, n-Propoxy oder iso-Propoxy steht, $R^7$ und $R^8$ gemeinsam mit dem Kohlenstoffatom, an das sie gebunden sind, für Cyclohexyl, Cyclopentyl oder Tetrahydrofuranyl stehen und m für die Zahl 0 oder 1 steht.

Insbesondere bevorzugt sind diejenigen Verbindungen der Formel (I), in denen $R^1$, $R^2$ und $R^3$ unabhängig voneinander jeweils für Wasserstoff oder Methyl stehen, A für eine CH-Gruppe oder ein Stickstoffatom steht, n für 0 steht, X für die Gruppen

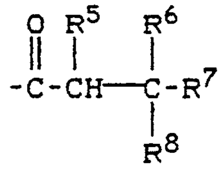 , 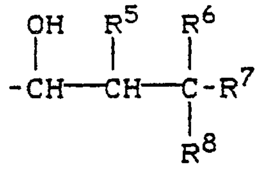 , 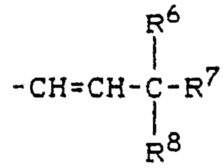 ,

(Ia) (Ib) (Ic)

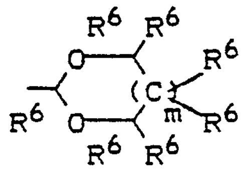 oder 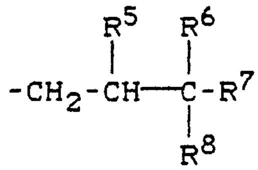

(Id) (Ie)

steht, wobei
$R^5$ für Wasserstoff und bei der Gruppe (Ie) zusätzlich für Hydroxyl steht,
$R^6$ und $R^7$ unabhängig voneinander jeweils für Wasserstoff oder Methyl stehen, wobei $R^6$ bei der Gruppe (Id) gleich oder verschieden sein kann, und
$R^8$ für Methyl oder tert.-Butyl steht und
m für die Zahl 0 oder 1 steht.

Verwendet man beispielsweise 4,6-Dimethyl-2-chlorpyrimidin und p-Neohexylthiophenol als Ausgangsstoffe, so kann der Verlauf des erfindungsgemäßen Verfahrens (A) durch das folgende Formelschema wiedergegeben werden:

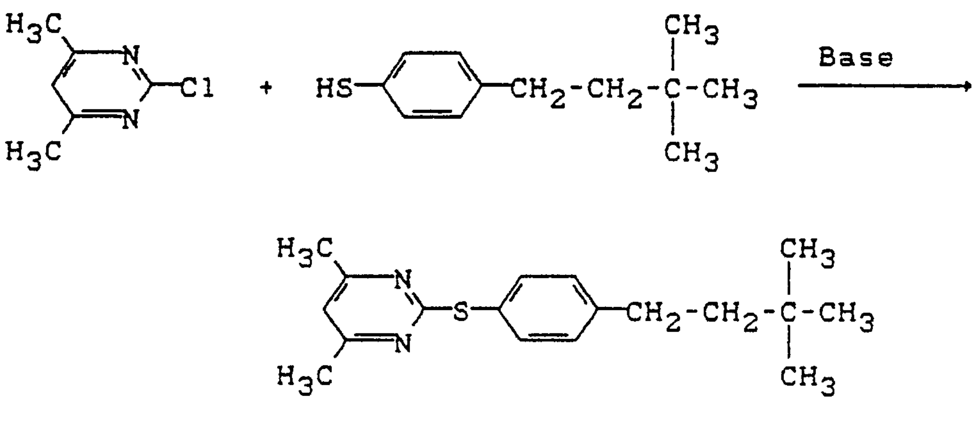


Verwendet man beispielsweise 4,5-Dimethyl-2-(4-neopentylcarbonyl-phenylthio)-pyrimidin und Natriumborhydrid als Ausgangsstoffe, so kann der Verlauf des erfindungsgemäßen Verfahrens (B) durch das folgende Formelschema wiedergegeben werden:

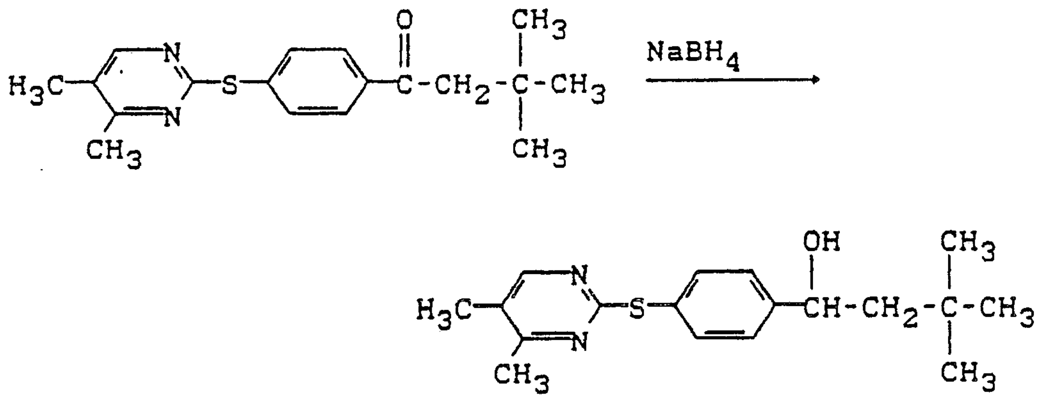


Verwendet man beispielsweise 4,5-Dimethyl-2-[4-(1-hydroxy)-neohexyl-phenylthio)-pyrimidin und Thionylchlorid in Gegenwart einer Base, so kann der Verlauf des erfindungsgemäßen Verfahrens (C) durch das folgende Formelschema wiedergegeben werden:

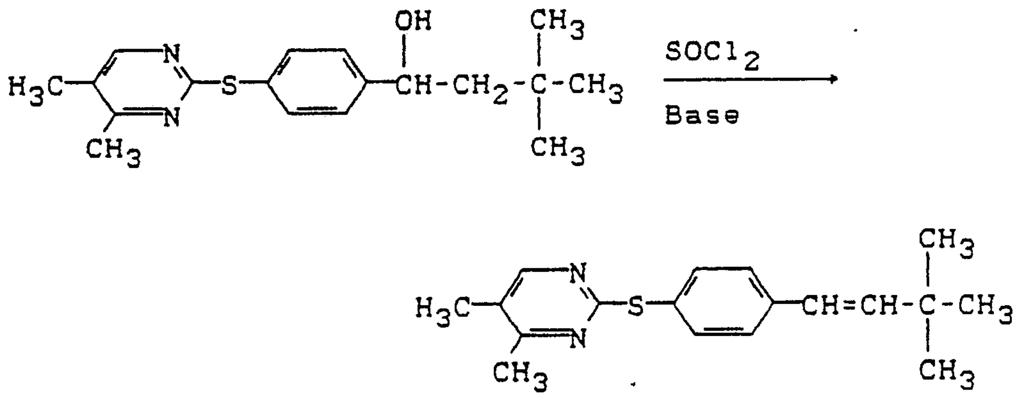


Verwendet man beispielsweise 4,6-Dimethyl-2-(4-acetophenylthio)-pyrimidin und Neopentylglykol, so kann der Verlauf des erfindungsgemäßen Verfahrens (D) durch das folgende Formelschema wiedergegeben werden:

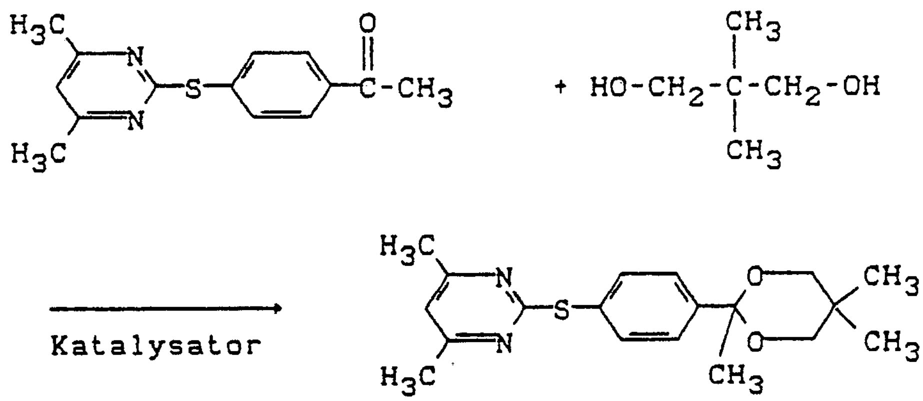


Die bei dem Verfahren (A) eingesetzten Thiophenole werden durch die allgemeine Formel (II) beschrieben. In ihr stehen die Reste $R^4$, X und n für die Reste, die schon bei der Beschreibung von Verbindungen der Formel (I) als bevorzugt bzw. besonders bevorzugt erwähnt wurden. Die Verbindungen der Formel (II) sind teilweise bekannt. Teilweise sind sie Gegenstand der eigenen Patentanmeldung DE-P 37 25 638.6 vom 3. August 1987 und DE-P 37 25 640.8 vom 3. August 1987.

Neu sind Thiophenole der Formel (IIz),

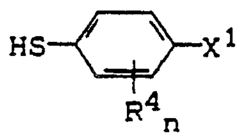

(IIz)

in welcher
$R^4$ für Halogen, Alkyl oder Alkoxy steht, wobei $R^4$ gleich oder verschieden sein kann,
n für eine Zahl 0, 1 oder 2 steht,
$X^1$ für die Gruppe

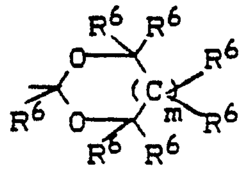

steht, wobei
$R^6$ gleich oder verschieden sein kann und jeweils für Wasserstoff oder Alkyl steht und
m für die Zahl 0 oder 1 steht.

Bevorzugt sind diejenigen Verbindungen der Formel (IIz), in welcher
$R^4$ für Halogen, $C_1$-$C_4$-Alkyl oder $C_1$-$C_4$-Alkoxy steht, wobei $R^4$ gleich oder verschieden sein kann,
n für eine Zahl 0, 1 oder 2 steht,
$X^1$ für die Gruppe

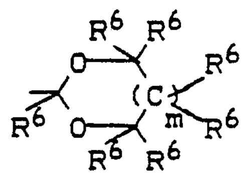

steht, wobei
$R^6$ gleich oder verschieden sein kann und jeweils für Wasserstoff oder $C_1$-$C_6$-Alkyl steht und
m für die Zahl 0 oder 1 steht.

Besonders bevorzugt sind die Verbindungen der Formel (IIz), in welcher
$R^4$ für Fluor, Chlor, Methyl, Ethyl, Methoxy oder Ethoxy steht,
n für die Zahl 0 oder 1 steht,
$X^1$ für die Gruppe

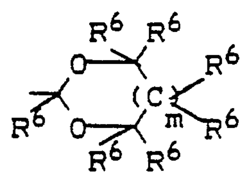

steht, wobei
$R^6$ gleich oder verschieden sein kann und jeweils für Wasserstoff oder $C_1$-$C_6$-Alkyl, insbesondere für Wasserstoff, Methyl, Ethyl oder iso-Propyl steht.

Man erhält beispielsweise Verbindungen der Formel (II), wenn man

E) Verbindungen der Formel (VII)

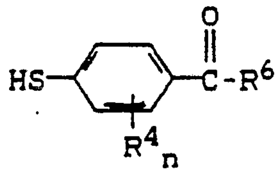

(VII)

in welcher
$R^4$, $R^6$ und n die oben angegebene Bedeutung haben,
mit Diolen der Formel (V)

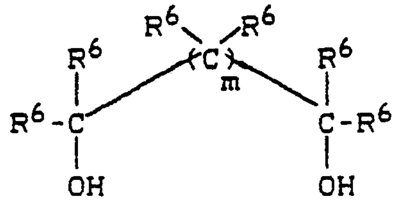

(V)

in welcher
$R^6$ und m die oben angegebene Bedeutung haben,
in Gegenwart eines Lösungsmittels wie beispielsweise Toluol
und in Gegenwart einer Säure wie beispielsweise Toluolsulfonsäure bei Temperaturen von 100 bis 120° C kondensiert.

Weiterhin erhält man beispielsweise Verbindungen der Formel (II), wenn man
F) Verbindungen der Formel (VI)

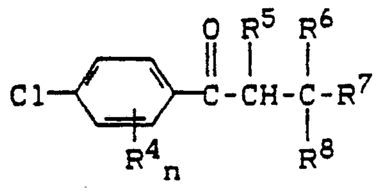

(VI)

in welcher
$R^4$, $R^5$, $R^6$, $R^7$, $R^8$ und n die oben angegebene Bedeutung haben,
mit Natriumsulfid oder Natriumhydrogensulfid in Gegenwart von Lösungsmitteln wie beispielsweise N-Methylpyrrolidon bei Temperaturen zwischen 100° C und 160° C umsetzt und aus dem dabei entstehenden Thiophenolat durch Zugabe von Säure das Thiophenol in Freiheit setzt,
oder wenn man
G) Verbindungen der allgemeinen Formel (VIII)

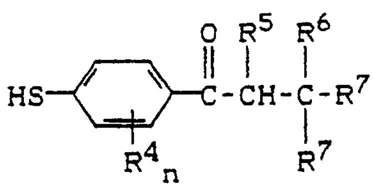

(VIII)

in welcher
$R^4$, $R^5$, $R^6$, $R^7$, $R^8$ und n die oben angegebene Bedeutung haben,
mit Reduktionsmitteln wie beispielsweise Natriumborhydrid und in Gegenwart eines Verdünnungsmittels wie beispielsweise Ethanol bei Temperaturen von 20° C bis +100° C, vorzugsweise 0° C bis 60° C, umsetzt,
oder wenn man
H) Aniline der Formel (IX)

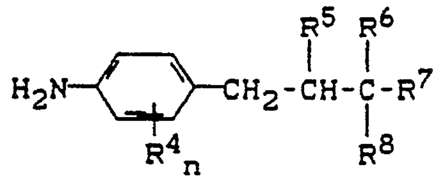

(IX)

in welcher
$R^4$, $R^5$, $R^6$, $R^7$, $R^8$ und n die oben angegebene Bedeutung haben,
mit salpetriger Säure in wäßriger Suspension, z.B. in verdünnter Salzsäure diazotiert, anschließend mit Natriumdisulfid umsetzt und die entstehenden Disulfide mit Reduktionsmitteln wie beispielsweise Zink in saurer Lösung bei Temperaturen zwischen 0 und 100 °C umsetzt.

Die bei den Verfahren (E), (F), (G) und (H) benötigten Verbindungen sind durch die Formeln (VI), (VII), (VIII) und (IX) allgemein definiert. In diesen Formeln haben die Reste bevorzugt bzw. besonders bevorzugt jeweils diejenigen Bedeutungen, die bereits im Zusammenhang mit der Beschreibung der Stoffe der Formel (I) bevorzugt bzw. besonders bevorzugt für diese Reste genannt wurden.

Die Verbindungen der Formeln (VI), (VII), (VIII) und (IX) sind bekannt oder lassen sich nach bekannten Verfahren herstellen.

Die bei dem obigen Verfahren (A) weiterhin als Ausgangsstoffe benötigten Pyri(mi)din-Derivate sind durch die Formel (III) definiert. In dieser Formel haben $R^1$, $R^2$, $R^3$ und A vorzugsweise diejenigen Bedeutungen, die bereits im Zusammenhang mit der Beschreibung der erfindungsgemäßen Stoffe der Formel (I) vorzugsweise für diese Reste genannt wurden. B steht vorzugsweise für Fluor, Chlor, Brom, $C_1$-$C_4$-Alkylsulfonyl, insbesondere Methylsulfonyl, Phenylsulfonyl oder 4-Methylphenylsulfonyl.

Die Pyri(mi)din-Derivate der Formel (III) sind bekannt oder lassen sich nach im Prinzip bekannten Methoden in einfacher Weise herstellen (vgl. DE-OS 2 101 359).

Als Säurebindemittel können bei der Durchführung des Verfahrens (A) alle üblicherweise für derartige Umsetzungen verwendbaren Säureakzeptoren verwendet werden. Vorzugsweise verwendbar sind Alkali- und Erdalkalioxide, -hydroxide und -carbonate, wie Natriumhydroxid, Kaliumhydroxid, Calciumoxid, Natriumcarbonat und Kaliumcarbonat, ferner Alkalialkoholate, -amide und -hydride, wie z.B. Natriummethylat, Natriumethylat, Kalium-tert.-butylat, Natriumamid und Natriumhydrid.

Als Verdünnungsmittel können bei der Durchführung des Verfahrens (A) alle üblichen inerten organischen Solventien verwendet werden. Vorzugsweise in Frage kommen Koh lenwasserstoffe, wie Benzin, Toluol und Xylol, ferner Ether, wie Dioxan, Glykoldimethylether und Diglykoldimethylether, außerdem Nitrile, wie Acetonitril, und auch stark polare Solventien, wie Dimethylsulfoxid, Sulfolan, Dimethylformamid und N-Methylpyrrolidon.

Die Reaktionstemperaturen können bei der Durchführung des Verfahrens (A) innerhalb eines größeren Bereiches variiert werden. Im allgemeinen arbeitet man bei Temperaturen zwischen 0 °C und 200 °C, vorzugsweise zwischen 50 °C und 150 °C.

Die Umsetzungen nach den Verfahren (A), (B), (C) und (D) werden im allgemeinen unter Normaldruck vorgenommen.

Bei der Durchführung des Verfahrens (A) setzt man die Ausgangsstoffe der Formeln (II) und (III) im allgemeinen in angenähert äquimolaren Mengen um. Es ist jedoch auch möglich, die eine oder andere Komponente in einem größeren Überschuß zu verwenden. Die Aufarbeitung erfolgt nach üblichen Methoden.

Die bei dem Verfahren (B) eingesetzten 4-Acyl-[2-pyri(mi)dyl]-thioarylether werden durch die allgemeine Formel (Ia) beschrieben und sind Gegenstand dieser Anmeldung. In ihr stehen die Reste $R^1$, $R^2$, $R^3$, $R^4$, $R^5$, $R^6$, $R^7$, $R^8$, A und n für die Reste, die schon bei der Beschreibung von Verbindungen der Formel (I) als bevorzugt bzw. besonders bevorzugt genannt wurden. Die Darstellung der Verbindungen der Formel (Ia) wird durch das Verfahren (A) beschrieben.

Als Reduktionsmittel können bei der Durchführung des Verfahrens (B) alle üblicherweise für derartige Umsetzungen verwendbaren komplexen Hydride verwendet werden. Vorzugsweise verwendbar sind Lithiumaluminiumhydrid, Dibutylaluminiumborhydrid, Lithiumborhydrid, Natriumborhydrid, Natriumcyanoborhydrid und Kaliumborhydrid.

Als Verdünnungsmittel können bei der Durchführung des Verfahrens (B) alle für derartige Reduktionen üblichen Solventien verwendet werden.

Vorzugsweise verwendbar sind Alkohole bzw. Ether, wie Methanol, Ethanol, Isopropanol, Cyclohexanol, Ethylenglykol, Ethylenglykolmonomethylether, Diethylenglykol, Diethylenglykolmonoethylether, Diethylether,

Tetrahydrofuran, Dioxan sowie Essigsäure.

Die Reaktionstemperaturen können bei der Durchführung des erfindungsgemäßen Verfahrens (B) innerhalb eines größeren Bereiches variiert werden. Im allgemeinen arbeitet man bei Temperaturen zwischen -20°C und +100°C, vorzugsweise zwischen 0°C und 60°C.

Bei der Durchführung des Verfahrens (B) setzt man die Ausgangsstoffe der Formel (Ia) und die Reduktionsmittel in angenähert äquimolaren Mengen um. Es ist jedoch auch möglich, die eine oder andere Komponente in einem größeren Überschuß zu verwenden. Die Aufarbeitung erfolgt nach üblichen Methoden.

Die bei dem Verfahren (C) eingesetzten Alkohole werden durch die allgemeine Formel (Ib) beschrieben und sind Gegenstand dieser Anmeldung. In ihr stehen die Reste $R^1$, $R^2$, $R^3$, $R^4$, $R^5$, $R^6$, $R^7$, $R^8$, A und n vorzugsweise bzw. besonders bevorzugt für die Reste, die schon bei der Beschreibung von Verbindungen der Formel (I) als bevorzugt bzw. besonders bevorzugt genannt wurden. Die Darstellung der Verbindungen der Formel (Ib) wird durch das Verfahren (B) beschrieben.

Als Halogenierungsmittel können bei der Durchführung des Verfahrens (C) alle üblicherweise für derartige Umsetzungen verwendbaren Nichtmetallhalogenide eingesetzt werden. Vorzugsweise verwendet man Thionylchlorid, Phosphortrichlorid, Phosphortribromid, Phosphoroxychlorid oder Phosphorpentachlorid.

Als Hilfsbasen verwendet man bei der Durchführung des Verfahrens (C) vorzugsweise organische Stickstoffbasen wie Pyridin, Picolin, Lutidin, Triethylamin, Tributylamin, Ethyl-diisopropylamin, Diazabicycloundecen (DBU) oder Diazabicyclo(4,3,0)non-5-en (DBN).

Als Verdünnungsmittel können bei Verfahren (C) alle gegen Halogenierungsmittel inerte Solventien verwendet werden. Vorzugsweise verwendbar sind Kohlenwasserstoffe wie Benzol, Toluol und Xylol, ferner Ether wie Diethylether, Tetrahydrofuran und Dioxan sowie halogenierte Kohlenwasserstoffe wie Methylenchlorid, Chloroform, Tetrachlorkohlenstoff und Ethylenchlorid. Weiterhin können auch die oben erwähnten Hilfsbasen als Verdünnungsmittel verwendet werden.

Die Reaktionstemperaturen können bei der Durchführung des Verfahrens (C) innerhalb eines größeren Bereiches variiert werden. Im allgemeinen arbeitet man bei Temperaturen zwischen -20°C und 150°C, vorzugsweise zwischen 0°C und 100°C.

Bei der Durchführung des Verfahrens (C) setzt man die Ausgangsstoffe der Formel (Ib), das Halogenierungsagenz sowie die Hilfsbase in angenähert äquimolaren Mengen um. Es ist jedoch auch möglich, die eine oder andere Komponente in einem größeren Überschuß zu verwenden. Die Aufarbeitung erfolgt nach üblichen Methoden.

Die bei dem Verfahren (D) als Zwischenprodukte eingesetzten 4-Carbonyl-[2-pyri(mi)dyl]-thioarylether werden durch die Formel (IV) beschrieben

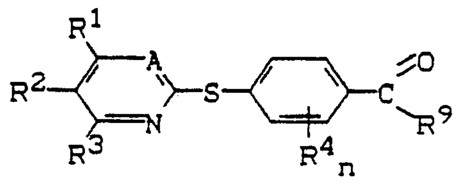

(IV).

In ihr stehen die Reste $R^1$, $R^2$, $R^3$, $R^4$, A und n vorzugsweise bzw. besonders bevorzugt für die Reste, die schon bei der Beschreibung von Verbindungen der allgemeinen Formel (I) als bevorzugt bzw. besonders bevorzugt erwähnt wurden. $R^9$ steht vorzugsweise für Wasserstoff oder $C_1$-$C_8$-Alkyl, besonders bevorzugt für Wasserstoff oder $C_1$-$C_6$-Alkyl.

Die 4-Carbonyl-[2-pyri(mi)dyl]-thioarylether der Formel (IV), in welcher die Reste $R^1$, $R^2$, $R^3$, $R^4$, A und n die obengenannte Bedeutung haben und $R^9$ für Wasserstoff steht sind neu und werden in der Formel (IVa) zusammengefaßt

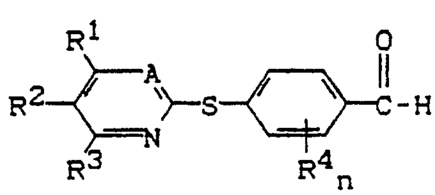

(IVa)

Verbindungen der Formel (IVa) werden erhalten, indem man 4-[2-Pyri(mi)dylthio]-benzylalkohole der

Formel (X)

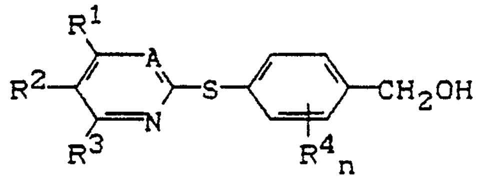

(X)

in welcher

$R^1$, $R^2$, $R^3$, $R^4$, A und n die oben angegebene Bedeutung haben,

in Gegenwart eines Verdünnungsmittels mit üblichen Oxidationsmitteln oxidiert.

Als Oxidationsmittel kommen dabei die für solche gezielten Oxidationen üblichen Reagentien in Frage, wie z.B. Chromtrioxid, Dichromate, Mangandioxid, Salpetersäure, Sauerstoff in Gegenwart von Übergangsmetallkomplexen, Cer-(IV)-Salze oder Dimethylsulfoxid in Gegenwart von Säurechloriden, Säureanhydriden, Chlor, Brom oder Carbodiimiden und Säuren. Die Oxidation mit Dimethylsulfoxid kann beispielsweise so durchgeführt werden, daß in einem inerten Lösungsmittel wie beispielsweise einem Halogenkohlenwasserstoff Dimethylsulfoxid mit Reagentien wie Acetanhydrid, Trifluoressigsäureanhydrid, Thionylchlorid, Oxalylchlorid, Chlor oder Schwefeltrioxid in Gegenwart von Pyridin aktiviert und dann mit dem Benzylalkohol der Formel (X) bei Temperaturen zwischen -80° C und +100° C umgesetzt wird.

Die Verbindungen der Formel (X) sind neu und können

I) durch Umsetzung von Pyri(mi)din-Derivaten der Formel (III)

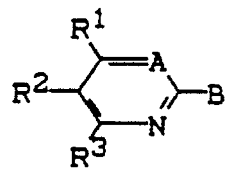

(III)

in welcher

$R^1$, $R^2$, $R^3$, A und B die oben angegebene Bedeutung haben,

mit Thiophenolen der Formel (XI)

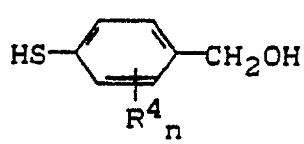

(XI)

in welcher

$R^4$ und n die oben angegebene Bedeutung haben,

nach den bei Verfahren (A) angegebenen Reaktionsbedingungen erhalten werden oder

K) durch Reduktion von 4-[2-pyri(mi)dylthio)-benzoesäureestern der Formel (XII)

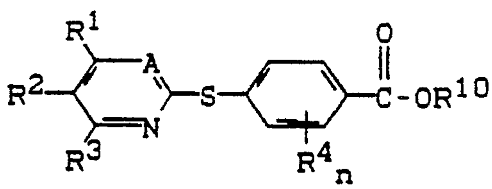

(XII)

in welcher

$R^1$, $R^2$, $R^3$, $R^4$, A und n die oben angegebene Bedeutung haben und

$R^{10}$ für $C_1$-$C_8$-Alkyl oder $C_3$-$C_8$-Cycloalkyl, insbesonders für $C_1$-$C_6$-Alkyl oder $C_3$-$C_6$-Cycloalkyl, steht, hergestellt werden.

Die Reduktion wird durch Umsetzung mit komplexen Hydriden wie z.B. Lithiumaluminiumhydrid und Natriumborhydrid mit oder ohne Zusatz von Lewis-Säure-Katalysatoren in inerten Lösungsmitteln wie Ethern oder Kohlenwasserstoffen bei Temperaturen zwischen -20 °C und +200 °C durchgeführt.

Die weiterhin zur Herstellung der Verbindungen der Formel (IV) benötigten Zwischenprodukte der Formeln (XI) und (XII) sind bekannt (vgl. J. Polymer Sci. 77, 221 (1955) und EP-A-226 837).

Die neuen 4-Carbonyl-[2-pyri(mi)dyl]-thioarylether der Formel (IV), in welcher $R^9$ für Alkyl steht und die übrigen Reste die bereits angegebene Bedeutung haben, erhält man, wenn man Thiophenole der Formel (XIII)

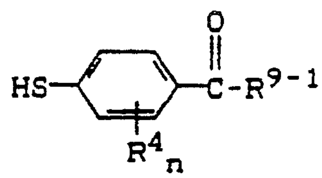

(XIII)

in welcher
$R^4$ und n die oben angegebene Bedeutung haben und
$R^{9-1}$ für Alkyl steht,
mit Pyri(mi)dyl-Derivaten der Formel (III)

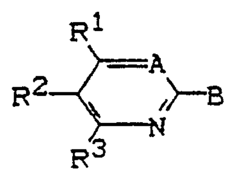

(III)

in welcher
$R^1$, $R^2$, $R^3$, A und B die oben angegebene Bedeutung haben,
gemäß den bei Verfahren (A) angegebenen Reaktionsbedingungen in Gegenwart eines Verdünnungsmittels und gegebenenfalls in Gegenwart eines Säurebindemittels umsetzt.

Die Thiophenole der Formel (XIII) sind bekannte Verbindungen der organischen Chemie.

Als Beispiele für 4-Acyl-(2-pyri(mi)dyl)-thioarylether der Formel (IV) seien genannt:
4-Acetyl-(2,5-dimethyl-2-pyridylthio)-benzol,
4-Acetyl-(4-methyl-2-pyrimidylthio)-benzol,
4-Acetyl-(4,5-dimethyl-2-pyrimidylthio)-benzol,
4-Acetyl-(4,6-dimethyl-2-pyrimidylthio)-benzol,
4-Acetyl-(trimethyl-2-pyrimidylthio)-benzol und
4-Acetyl-(5-ethyl-4-methyl-2-pyrimidylthio)-benzol.

Die bei dem Verfahren (D) als weitere Komponente benutzten Diole der Formel (IV) sind bekannt oder lassen sich nach üblichen Methoden in einfacher Weise herstellen.

Als Katalysator können bei der Durchführung des Verfahrens (D) alle üblicherweise für derartige Umsetzungen verwendbaren Katalysatoren verwendet werden. Vorzugsweise verwendbar sind anorganische Säuren wie gasförmige Chlorwasserstoffsäure oder konz. Schwefelsäure und organische Säuren wie p-Toluolsulfonsäure, Methansulfonsäure sowie Trifluormethansulfonsäure. Außerdem verwendbar sind z.B. Pyridinium- und Alkylpyridiniumsalze der p-Toluolsulfonsäure, aber auch anorganische Salze mit Lewis-Säurecharakter wie z.B. Zinkchlorid, Eisen-III-chlorid und geglühtes Kupfer-II-sulfat.

Weiterhin können wasserentziehende Agenzien zugegeben werden. Die für derartige Reaktionen verwendeten wasserentziehenden Agenzien sind: Orthoameisensäureethylester, -methylester, Orthoessigsäureethylester, - methylester und anorganische wasserfreie Salze wie geglühtes Magnesiumsulfat. Es ist aber auch möglich Molekularsiebe zu verwenden.

Als Verdünnungsmittel können bei der Durchführung des Verfahrens (D) alle üblichen inerten organischen Solven tien verwendet werden. Vorzugsweise in Frage kommen Kchlenwasserstoffe wie Benzin, Benzol, Toluol, Xylol, Cyclohexan und Petrolether.

Die Reaktionstemperaturen können bei der Durchführung des Verfahrens (D) innerhalb eines größeren Bereiches variiert werden. Im allgemeinen arbeitet man bei Temperaturen zwischen 0 °C und 200 °C, vorzugsweise zwischen 20 °C und 150 °C.

Bei der Durchführung des Verfahrens (D) setzt man die Ausgangsstoffe der Formel (IV) und (V) im allgemeinen in angenähert äquimolaren Mengen um. Es ist jedoch auch möglich, die eine oder andere Komponente in einem größeren Überschuß zu verwenden. Die Aufarbeitung erfolgt nach üblichen Methoden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen:

Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen:

Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen:

Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen:

Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz, Obst-, Wein-, Citrus-, Nuß-, Bananen-, Kaffee-, Tee-, Gummi-, Ölpalm-, Kakao-, Beerenfrucht-und Hopfenanlagen und zur selektiven Unkrautbekämpfung in einjährigen Kulturen eingesetzt werden.

Dabei lassen sich die erfindungsgemäßen Wirkstoffe mit besonders gutem Erfolg zur selektiven Bekämpfung von dikotylen Unkräutern in monokotylen Kulturen, wie beispielsweise Weizen, einsetzen, insbesondere im Nachauflaufverfahren.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie

Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; aus Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. Aryloxyalkancarbonsäuren wie 2,4 D; 2,4 DP, 2,4 DB; MCPA; MCPP oder Trichlorpyr; Aryloxy-phenoxy-alkansäureester wie Diclofop-methyl oder Fenoxaprop; Dinitroaniline wie Pendimethalin; Diphenylether wie Bifenox; Harnstoffe wie Chlortoluron, Isoproturon oder Methabenzthiazuron; Imidazolinone wie Imazamethabenz; Nitrile wie Bromoxynil oder Ioxynil; Sulfonylharnstoffe wie Chlorsulfuron, Metsulfuron oder Thiameturon; Triazindione wie Amethydione; Triazine wie Terbutryne; Triazinone wie Ethiozin oder Metribuzin; Bentazone oder Pyridate in Frage.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

## Herstellungsbeispiele

### Beispiel 1

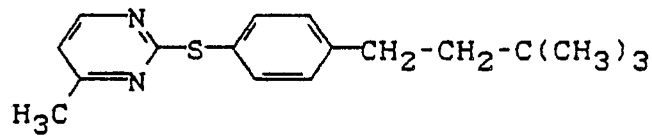

Verfahren A

4,36 g (20 mol) 4-Neohexyl-thiophenol werden in 20 ml N-Methylpyrrolidon under Stickstoffatmosphäre vorgelegt, mit 1,35 g (24 mmol) gepulvertem Kaliumhydroxid versetzt, 15 Minuten verrührt und nach Zugabe von 2,56 g (20 mmol) 4-Methyl-2-chlorpyrimidin unter Dünnschicht-Kontrolle auf 110 °C erwärmt. Anschließend wird die Reaktionsmischung in 100 ml Wasser gegossen, fünfmal mit n-Hexan extrahiert, getrocknet und im Wasserstrahlvakuum einrotiert. Nach der Säulenchromatographie (Laufmittel: Cyclohexan/Essigester 3:1) und anschließender Kristallisation aus n-Hexan erhält man 3,13 g (54,6 % der Theorie) 4-Neohexyl-(4-methyl-2-pyrimidyl)-thiophenylether vom Schmelzpunkt 32 °C.

Beispiel 2

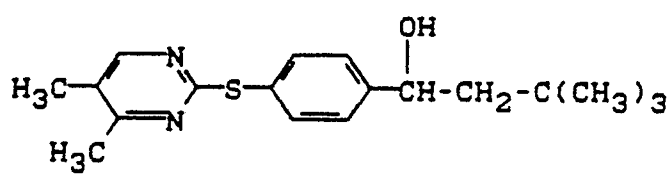

Verfahren B

62,4 g (0,2 Mol) 4-Neopentylcarbonyl-(4,5-dimethyl-2-pyrimidylthio)-benzol werden in 400 ml Ethanol suspendiert und innerhalb von 5 Minuten werden 3,8 g (0,1 Mol) Natriumborhydrid portionsweise zugefügt. Nach 2 Stunden wird das Lösungsmittel im Wasserstrahlvakuum abrotiert, der Rückstand in Methylenchlorid aufgenommen, mit Wasser gewaschen, getrocknet und erneut im Wasserstrahlvakuum einrotiert. Nach Umkristallisieren aus Toluol/n-Hexan erhält man 47,1 g (74,5 % der Theorie) 4-(1-Hydroxy-neohexyl)-(4,5-dimethyl-2-pyrimidylthio)-benzol vom Schmelzpunkt 85 °C.

Beispiel 3

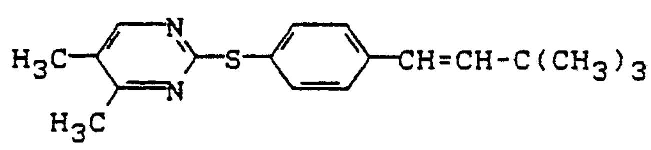

Verfahren C

23,75 g (75 mmol) 4-(1-Hydroxy-1-neohexyl)-(4,5-dimethyl-2-pyrimidylthio)-benzol werden in 18,2 ml (0,225 Mol) absolutem Pyridin vorgelegt. Bei -10 °C bis 0 °C werden 11 ml (0,15 Mol) Thionylchlorid zugetropft und anschließend auf 105 °C unter Dünnschicht-Kontrolle erwärmt. Zu der Mischung tropft man 250 ml Wasser, extrahiert dreimal mit n-Hexan, trocknet und rotiert im Wasserstrahlvakuum ein. Der Rückstand wird an Kieselgel mit Cyclohexan/Essigester 3:1 chromatographiert und mit Essigester/n-Hexan zur Kristallisation gebracht.

Man erhält 4,83 g (21,6 % der Theorie) des gewünschten Produktes vom Schmelzpunkt 100 °C.

Beispiel 4

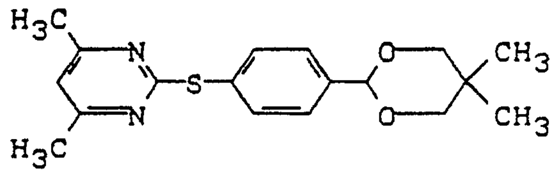

Verfahren D

4,4 g (20 mmol) 4-(4,6-Dimethyl-2-pyrimidylthio)-benzaldehyd, 6,25 g (60 mmol) Neopentylglykol und 30 mg p-Toluolsulfonsäure werden in 150 ml Toluol am Wasserabscheider unter Dünnschicht-Kontrolle rückflussiert. Nach Beendigung der Reaktion wird das Reaktionsgemisch in gesättigte Natriumhydrogencarbonatlösung eingerührt, die Toluolphase abgetrennt, getrocknet und im Wasserstrahlvakuum einrotiert. Durch Umkristallisieren aus Ether/n-Hexan erhält man 3,84 g (60,3 % der Theorie) 4-[2-(5,5-Dimethyl-1,3-dioxanyl)]-(4,6-dimethyl-2-pyrimidylthio)-benzol vom Schmelzpunkt 93 °C.

In entsprechender Weise und gemäß den allgemeinen Angaben zur Herstellung erhält man die in den folgenden Tabellen aufgeführten 2-Pyri(mi)dyl-thioarylether der Formel (I)

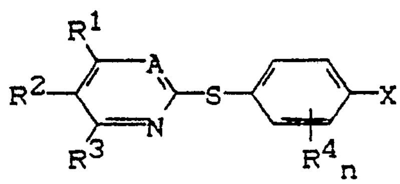

(I)

**Tabelle 1a: Verbindungen der Formel (Ia)**

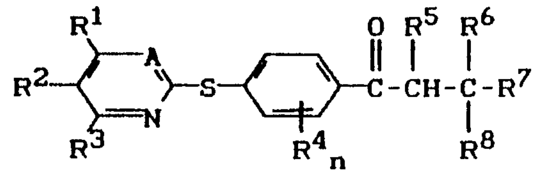

(Ia)

| Bsp.-Nr. | $R^1$ | $R^2$ | $R^3$ | A | $R^4$ | n | $R^5$ | $R^6$ | $R^7$ | $R^8$ | Schmelzpunkt (°C) |
|---|---|---|---|---|---|---|---|---|---|---|---|
| 5 | $CH_3$ | H | $CH_3$ | CH | - | 0 | H | H | H | $C(CH_3)_3$ | |
| 6 | $CH_3$ | H | H | CH | - | 0 | H | $CH_3$ | $CH_3$ | $C(CH_3)_3$ | |
| 7 | $CH_3$ | H | H | N | - | 0 | H | $CH_3$ | $CH_3$ | $CH_3$ | $n_D^{20}$ : 1,592 |
| 8 | $CH_3$ | $CH_3$ | H | N | - | 0 | H | H | H | $C(CH_3)_3$ | Öl |
| 9 | $CH_3$ | $CH_3$ | H | N | - | 0 | H | H | $CH_3$ | $CH_3$ | |
| 10 | $CH_3$ | $CH_3$ | H | N | - | 0 | H | $CH_3$ | $CH_3$ | $CH_3$ | $n_D^{20}$ : 1,5903 |
| 11 | $CH_3$ | H | $CH_3$ | N | - | 0 | $CH_3$ | H | H | $CH_3$ | Öl |
| 12 | $CH_3$ | H | $CH_3$ | N | - | 0 | H | H | H | $C(CH_3)_3$ | 54 |
| 13 | $CH_3$ | H | $CH_3$ | N | - | 0 | H | H | $CH_3$ | $CH_3$ | $n_D^{20}$ : 1,5923 |
| 14 | $CH_3$ | H | $CH_3$ | N | - | 0 | H | $CH_3$ | $CH_3$ | $CH_3$ | Öl |
| 15 | $CH_3$ | $CH_3$ | $CH_3$ | N | - | 0 | H | H | H | $C(CH_3)_3$ | 83 |
| 16 | $CH_3$ | $CH_3$ | $CH_3$ | N | - | 0 | H | H | $CH_3$ | $CH_3$ | |
| 17 | $CH_3$ | $CH_3$ | $CH_3$ | N | - | 0 | H | $CH_3$ | $CH_3$ | $CH_3$ | 96-98 |
| 18 | $CH_3$ | $C_2H_5$ | H | N | - | 0 | H | $CH_3$ | $CH_3$ | $CH_3$ | |

Tabelle 1b: Verbindungen der Formel (Ib)

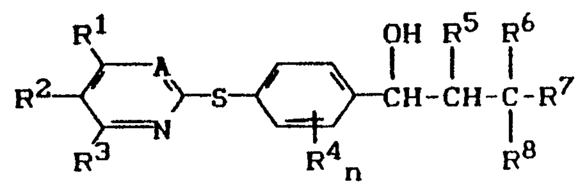

(Ib)

| Bsp.-Nr. | $R^1$ | $R^2$ | $R^3$ | A | $R^4$ | n | $R^5$ | $R^6$ | $R^7$ | $R^8$ | Schmelzpunkt (°C) |
|---|---|---|---|---|---|---|---|---|---|---|---|
| 19 | $CH_3$ | H | $CH_3$ | CH | - | 0 | H | $CH_3$ | $CH_3$ | $CH_3$ | |
| 20 | $CH_3$ | H | H | N | - | 0 | H | $CH_3$ | $CH_3$ | $CH_3$ | |
| 21 | $CH_3$ | H | $CH_3$ | N | - | 0 | H | $CH_3$ | $CH_3$ | $CH_3$ | 71 |
| 22 | $CH_3$ | $CH_3$ | $CH_3$ | N | - | 0 | H | $CH_3$ | $CH_3$ | $CH_3$ | |
| 23 | $CH_3$ | $C_2H_5$ | H | N | - | 0 | H | $CH_3$ | $CH_3$ | $CH_3$ | |

**Tabelle 1c: Verbindungen der Formel (Ic)**

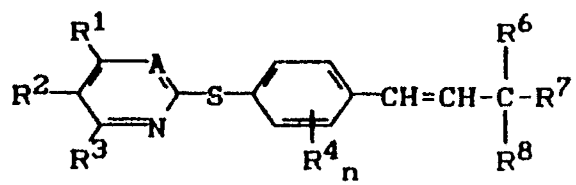

(Ic)

| Bsp.-Nr. | $R^1$ | $R^2$ | $R^3$ | A | $R^4$ | n | $R^6$ | $R^7$ | $R^8$ | Schmelzpunkt (°C) |
|---|---|---|---|---|---|---|---|---|---|---|
| 24 | $CH_3$ | H | $CH_3$ | CH | - | 0 | $CH_3$ | $CH_3$ | $CH_3$ | |
| 25 | $CH_3$ | H | H | N | - | 0 | $CH_3$ | $CH_3$ | $CH_3$ | |
| 26 | $CH_3$ | H | $CH_3$ | N | - | 0 | $CH_3$ | $CH_3$ | $CH_3$ | Öl |
| 27 | $CH_3$ | $CH_3$ | $CH_3$ | N | - | 0 | $CH_3$ | $CH_3$ | $CH_3$ | |
| 28 | $CH_3$ | $C_2H_5$ | H | N | - | 0 | $CH_3$ | $CH_3$ | $CH_3$ | |

**Tabelle 1d: Verbindungen der Formel (Id)**

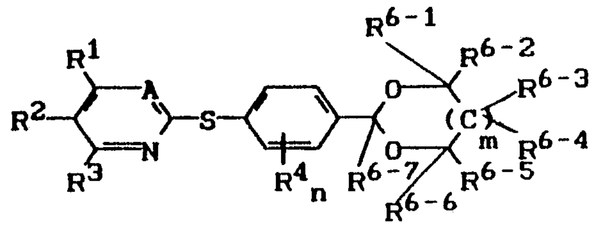

(Id)

| Bsp. Nr. | $R^1$ | $R^2$ | $R^3$ | A | $R^4$ | n | $R^{6-1}$ | $R^{6-2}$ | $R^{6-3}$ | $R^{6-4}$ | $R^{6-5}$ | $R^{6-6}$ | $R^{6-7}$ | m | Schmelzpunkt (°C) |
|---|---|---|---|---|---|---|---|---|---|---|---|---|---|---|---|
| 29 | $CH_3$ | H | $CH_3$ | CH | - | 0 | $CH_3$ | $CH_3$ | - | - | H | H | H | 0 | |
| 30 | $CH_3$ | H | $CH_3$ | CH | - | 0 | $CH_3$ | $CH_3$ | - | - | H | H | $CH_3$ | 0 | |
| 31 | $CH_3$ | H | $CH_3$ | CH | - | 0 | $CH_3$ | $CH_3$ | H | H | H | H | H | 1 | |
| 32 | $CH_3$ | H | $CH_3$ | CH | - | 0 | $CH_3$ | $CH_3$ | H | H | H | $CH_3$ | H | 1 | |
| 33 | $CH_3$ | $CH_3$ | H | N | - | 0 | $CH_3$ | $CH_3$ | - | - | H | H | H | 0 | |
| 34 | $CH_3$ | $CH_3$ | H | N | - | 0 | $CH_3$ | $CH_3$ | - | - | H | H | $CH_3$ | 0 | 63 |
| 35 | $CH_3$ | $CH_3$ | H | N | - | 0 | $CH_3$ | $CH_3$ | H | H | H | H | H | 1 | |
| 36 | $CH_3$ | $CH_3$ | H | N | - | 0 | $CH_3$ | $CH_3$ | H | H | H | $CH_3$ | H | 1 | |
| 37 | $CH_3$ | H | $CH_3$ | N | - | 0 | $CH_3$ | $CH_3$ | - | - | H | H | H | 0 | 53 |
| 38 | $CH_3$ | H | $CH_3$ | N | - | 0 | $CH_3$ | $CH_3$ | - | - | H | H | $CH_3$ | 0 | 70 |
| 39 | $CH_3$ | H | $CH_3$ | N | - | 0 | $CH_3$ | $CH_3$ | H | H | H | H | H | 1 | |
| 40 | $CH_3$ | H | $CH_3$ | N | - | 0 | $CH_3$ | $CH_3$ | H | H | H | $CH_3$ | H | 1 | 86 |
| 41 | $CH_3$ | H | $CH_3$ | N | - | 0 | H | H | $CH_3$ | $CH_3$ | H | H | $CH_3$ | 1 | 95 |

**Tabelle 1d: Verbindungen der Formel (Id)** (Fortsetzung)

| Bsp. Nr. | $R^1$ | $R^2$ | $R^3$ | A | $R^4$ | n | $R^{6-1}$ | $R^{6-2}$ | $R^{6-3}$ | $R^{6-4}$ | $R^{6-5}$ | $R^{6-6}$ | $R^{6-7}$ | m | Schmelzpunkt (°C) |
|---|---|---|---|---|---|---|---|---|---|---|---|---|---|---|---|
| 42 | $CH_3$ | $CH_3$ | $CH_3$ | N | - | 0 | $CH_3$ | $CH_3$ | - | - | H | H | H | 0 | |
| 43 | $CH_3$ | $CH_3$ | $CH_3$ | N | - | 0 | $CH_3$ | $CH_3$ | - | - | H | H | $CH_3$ | 0 | 90 |
| 44 | $CH_3$ | $CH_3$ | $CH_3$ | N | - | 0 | $CH_3$ | $CH_3$ | H | H | H | H | H | 1 | |
| 45 | $CH_3$ | $CH_3$ | $CH_3$ | N | - | 0 | $CH_3$ | $CH_3$ | H | H | H | $CH_3$ | H | 1 | |
| 46 | $CH_3$ | $C_2H_5$ | H | N | - | 0 | $CH_3$ | $CH_3$ | - | - | H | H | H | 0 | |
| 47 | $CH_3$ | $C_2H_5$ | H | N | - | 0 | $CH_3$ | $CH_3$ | - | - | H | H | $CH_3$ | 0 | |
| 48 | $CH_3$ | $C_2H_5$ | H | N | - | 0 | $CH_3$ | $CH_3$ | H | H | H | H | H | 1 | |
| 49 | $CH_3$ | $C_2H_5$ | H | N | - | 0 | $CH_3$ | $CH_3$ | H | H | H | $CH_3$ | H | 1 | |

**Tabelle 1e: Verbindungen der Formel (Ie)**

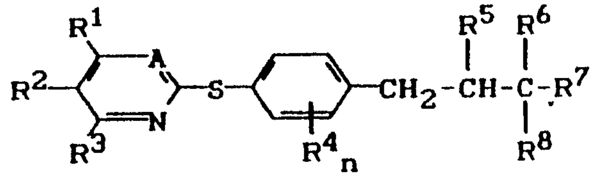

(Ie)

| Bsp.-Nr. | $R^1$ | $R^2$ | $R^3$ | A | $R^4$ | n | $R^5$ | $R^6$ | $R^7$ | $R^8$ | Schmelzpunkt (°C) |
|---|---|---|---|---|---|---|---|---|---|---|---|
| 50 | $CH_3$ | H | $CH_3$ | CH | - | 0 | H | H | H | $C(CH_3)_3$ | |
| 51 | $CH_3$ | H | $CH_3$ | CH | - | 0 | H | H | $CH_3$ | $CH_3$ | Öl |
| 52 | $CH_3$ | H | $CH_3$ | CH | - | 0 | H | $CH_3$ | $CH_3$ | $CH_3$ | Öl |
| 53 | $CH_3$ | $CH_3$ | H | N | - | 0 | H | H | H | $CH(CH_3)_2$ | |
| 54 | $CH_3$ | $CH_3$ | H | N | - | 0 | H | H | H | $C(CH_3)_3$ | 65 |
| 55 | $CH_3$ | $CH_3$ | H | N | - | 0 | H | H | $CH_3$ | $CH_3$ | 34 |
| 56 | $CH_3$ | $CH_3$ | H | N | - | 0 | H | $CH_3$ | $CH_3$ | $CH_3$ | 68 |
| 57 | $CH_3$ | H | $CH_3$ | N | - | 0 | H | H | H | $C(CH_3)_3$ | Öl |
| 58 | $CH_3$ | H | $CH_3$ | N | - | 0 | H | H | $CH_3$ | $CH_3$ | Öl |
| 59 | $CH_3$ | H | $CH_3$ | N | - | 0 | H | $CH_3$ | $CH_3$ | $CH_3$ | Öl |
| 60 | $CH_3$ | $CH_3$ | $CH_3$ | N | - | 0 | H | H | H | $C(CH_3)_3$ | Öl |
| 61 | $CH_3$ | $CH_3$ | $CH_3$ | N | - | 0 | H | H | $CH_3$ | $CH_3$ | Öl |

**Tabelle 1e: Verbindungen der Formel (Ie)** (Fortsetzung)

| Bsp.-Nr. | $R^1$ | $R^2$ | $R^3$ | A | $R^4$ | n | $R^5$ | $R^6$ | $R^7$ | $R^8$ | Schmelzpunkt (°C) |
|---|---|---|---|---|---|---|---|---|---|---|---|
| 62 | $CH_3$ | $CH_3$ | $CH_3$ | N | - | 0 | H | $CH_3$ | $CH_3$ | $CH_3$ | 53 |
| 63 | $CH_3$ | $C_2H_5$ | H | N | - | 0 | H | H | H | $C(CH_3)_3$ | |
| 64 | $CH_3$ | $C_2H_5$ | H | N | - | 0 | H | H | $CH_3$ | $CH_3$ | Öl |
| 65 | $CH_3$ | $C_2H_5$ | H | N | - | 0 | H | $CH_3$ | $CH_3$ | $CH_3$ | 34 |
| 66 | $CH_3$ | H | $CH_3$ | CH | - | 0 | H | $CH_3$ | $CH_3$ | $CH_3$ | |
| 67 | $CH_3$ | $CH_3$ | H | N | - | 0 | H | $CH_3$ | $CH_3$ | $CH_3$ | 88 |
| 68 | $CH_3$ | H | $CH_3$ | N | - | 0 | H | $CH_3$ | $CH_3$ | $CH_3$ | 91 |
| 69 | $CH_3$ | $CH_3$ | $CH_3$ | N | - | 0 | H | $CH_3$ | $CH_3$ | $CH_3$ | |
| 70 | $CH_3$ | $C_2H_5$ | H | N | - | 0 | H | $CH_3$ | $CH_3$ | $CH_3$ | |

Herstellungsbeispiele für Zwischenprodukte:

Beispiel IV-1:

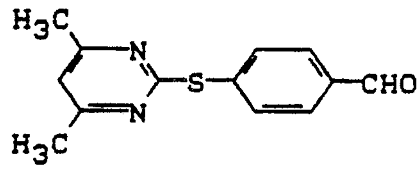

Unter Stickstoff werden 20,5 ml (0,22 Mol) Oxalylchlorid in 400 ml Dichlormethan vorgelegt und bei -60 °C 34 g (0,44 Mol) Dimethylsulfoxid in 100 ml Dichlormethan zugetropft. Dann werden bei -30 °C 24,6 g (0,1 Mol) 4-(4,6-Dimethyl-2-pyrimidylthio)-benzylalkohol, gelöst in 100 ml Dichlormethan, zugetropft. Es wird 15 Minuten nachgerührt, dann bei -30 °C 101 g (1 Mol) Triethylamin zugefügt und langsam auf Raumtemperatur erwärmt. Dann wird auf 1000 ml Eiswasser gegossen, die organische Phase abgetrennt, mit Wasser gewaschen und mit Natriumsulfat getrocknet. Beim Einengen bleiben 33 g eines Festkörpers zurück, der aus Petrolether/Dichlormethan umkristallisiert wird. Das zunächst ausfallende Öl wird abgetrennt, dann fallen 16 g Kristalle des gewünschten Produkts vom Schmelzpunkt 87-90 °C aus. Aus dem Öl und der Mutterlauge können durch Chromatographie mit Essigester/Petrolether 1:2 auf Kieselgel (RF 0,43) weitere 3 g Produkt isoliert werden. Die Gesamtausbeute beträgt 78 % der Theorie.

Beispiel (X-1)

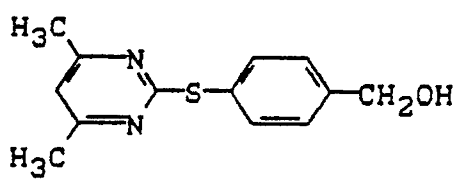

a) aus 4-Mercaptobenzylalkohol

1,6 g (30 mmol) Kaliumhydroxidpulver werden in 30 ml N-Methylpyrrolidon suspendiert, 4,9 g (35 mmol) 4-Mercaptobenzylalkohol als Lösung in 10 ml N-Methyl-pyrrolidon unter Stickstoff zugetropft und dann 4,2 g (30 mmol) 2-Chlor-4,4-dimethyl-pyrimidin eingetragen. Dann wird 1 Stunde bei 120 °C gerührt, das Lösungsmittel bei 0,1 mbar bis 80 °C Innentemperatur abdestilliert, der Rückstand auf Eiswasser gegossen und mit Chloroform extrahiert. Der Extrakt wird über Natriumsulfat getrocknet, eingeengt und der Rückstand (7,9 g) mit Essigester/Petrolether 1:1 (RF = 0,38) auf Kieselgel chromatographiert.
Ausbeute: 4,4 g (60 % der Theorie), Fp.: 87-91 °C.

b) durch Reduktion

3,7 g (0,1 Mol) Lithiumaluminiumhydrid werden in 100 ml absolutem Tetrahydrofuran unter Stickstoff vorgelegt und 28,8 g (0,1 Mol) 4-(4,6-Dimethyl-2- pyrimidylthio)-benzylalkohol als Lösung in 100 ml absolutem Tetrahydrofuran ohne äußere Kühlung zugetropft. Es wird 3 Stunden bei Raumtemperatur nachgerührt und über Nacht stehengelassen. Dann werden 3,7 ml Wasser in 10 ml Tetrahydrofuran, 3,7 ml 10 %ige Kaliumhydroxid-Lösung sowie 11,1 ml Wasser zugetropft. Der Festkörper wird abgesaugt, mit Tetrahydrofuran gewaschen, das Filtrat eingeengt und der Rückstand (22,7 g) wie oben chromatographiert.
Ausbeute: 17,4 g (71 % der Theorie), Fp.: 85-87 °C.

Anwendungsbeispiel

Beispiel A

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:
0 % = keine Wirkung (wie unbehandelte Kontrolle)
100 % = totale Vernichtung
In diesem Test zeigen beispielsweise die Verbindungen 7, 10, 17, 26, 40, 52, 56, 59 und 62 sowohl eine sehr gute herbizide Wirkung gegen Unkräuter, wie z.B. Amaranthus, Chenopodium oder Portulak, als auch eine sehr gute Nutzpflanzenverträglichkeit, z.B. in Weizen.

## Ansprüche

1. 2-Pyri(mi)dyl-thioarylether der Formel (I)

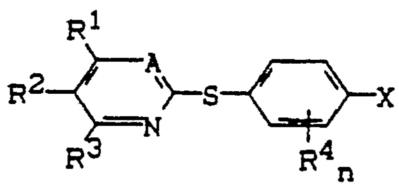

(I)

in welcher
$R^1$, $R^2$ und $R^3$ unabhängig voneinander jeweils für Wasserstoff oder Alkyl stehen,
A für eine CH-Gruppe oder ein Stickstoffatom steht,
$R^4$ für Halogen, Alkyl oder Alkoxy steht, wobei $R^4$ gleich oder verschieden sein kann,
n für eine Zahl 0, 1 oder 2 steht und
X für die Gruppen

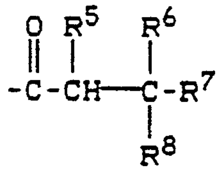 ,

(Ia)

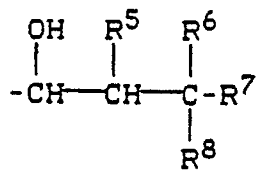 ,

(Ib)

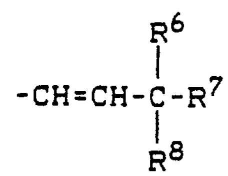 ,

(Ic)

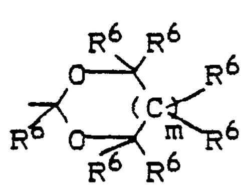

(Id)

oder

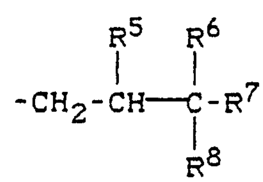

(Ie)

steht, wobei
$R^5$ für Wasserstoff, Alkyl, Halogenalkyl oder Alkoxy steht und bei der Gruppe (Ie) auch für Hydroxyl steht,
$R^6$ und $R^7$ unabhängig voneinander jeweils für Wasserstoff oder Alkyl stehen und wobei $R^6$ in der Gruppe (Id) jeweils gleich oder verschieden sein kann,
$R^8$ für Alkyl, Halogenalkyl oder Alkoxy steht oder
$R^7$ und $R^8$ gemeinsam mit dem Kohlenstoffatom, an das sie gebunden sind, für einen fünf-oder sechsgliedrigen carbocyclischen oder heterocyclischen Ring stehen und
m für die Zahl 0 oder 1 steht.

2. Verfahren zur Herstellung von 2-Pyri(mi)dyl-thioarylether der Formel (I)

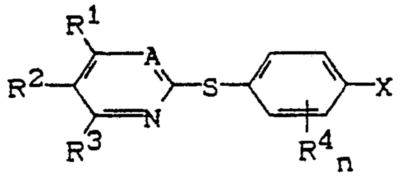

(I)

in welcher
$R^1$, $R^2$ und $R^3$ unabhängig voneinander jeweils für Wasserstoff oder Alkyl stehen,
A für eine CH-Gruppe oder ein Stickstoffatom steht,
$R^4$ für Halogen, Alkyl oder Alkoxy steht, wobei $R^4$ gleich oder verschieden sein kann,
n für eine Zahl 0, 1 oder 2 steht und
X für die Gruppen

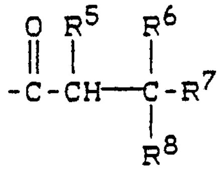

(Ia)

,

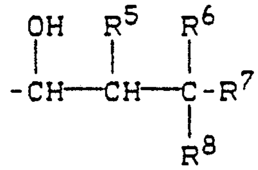

(Ib)

,

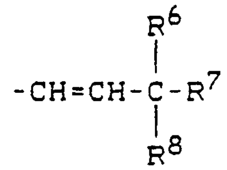

(Ic)

,

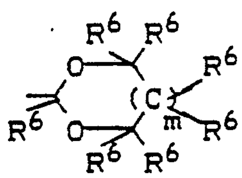

(Id)

oder

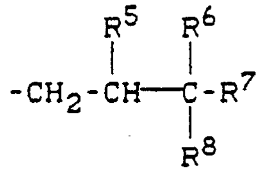

(Ie)

steht, wobei
$R^5$ für Wasserstoff, Alkyl, Halogenalkyl oder Alkoxy steht und bei der Gruppe (Ie) auch für Hydroxyl steht,
$R^6$ und $R^7$ unabhängig voneinander jeweils für Wasserstoff oder Alkyl stehen und wobei $R^6$ in der Gruppe (Id) jeweils gleich oder verschieden sein kann,
$R^8$ für Alkyl, Halogenalkyl oder Alkoxy steht oder
$R^7$ und $R^8$ gemeinsam mit dem Kohlenstoffatom, an das sie gebunden sind, für einen fünf-oder sechsgliedrigen carbocyclischen oder heterocyclischen Ring stehen und
m für die Zahl 0 oder 1 steht,
dadurch gekennzeichnet, daß man

(A) Thiophenol-Derivate der Formel (II)

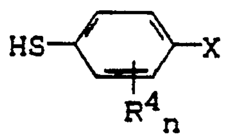

(II)

in welcher
$R^4$, X und n die oben angegebene Bedeutung haben,
mit Pyri(mi)dyl-Derivaten der Formel (III)

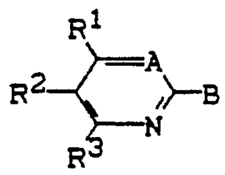

(III)

in welcher
$R^1$, $R^2$, $R^3$ und A die oben angegebene Bedeutung haben und
B für Halogen, Alkylsulfonyl oder gegebenenfalls substituiertes Arylsulfonyl steht,
in Gegenwart eines Verdünnungsmittels und gegebenenfalls in Gegenwart eines Säurebindemittels umsetzt,
oder daß man

B) 4-Acyl-[2-pyri(mi)dyl]-thioarylether der Formel (Ia)

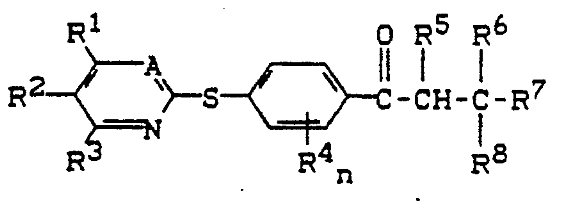

(Ia)

in welcher
$R^1$, $R^2$, $R^3$, $R^4$, $R^5$, $R^6$, $R^7$, $R^8$, A und n die oben angegebene Bedeutung haben,
zu Verbindungen der Formel (Ib)

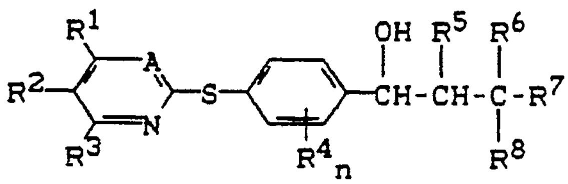

(Ib)

in welcher
$R^1$, $R^2$, $R^3$, $R^4$, $R^5$, $R^6$, $R^7$, $R^8$, A und n die oben angegebene Bedeutung haben,
in Gegenwart eines Verdünnungsmittels reduziert,
oder daß man

C) Verbindungen der Formel (Ib)

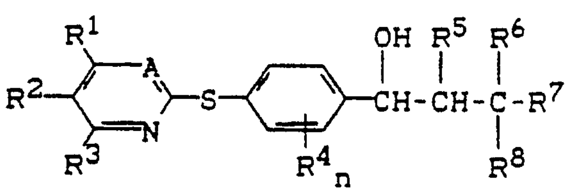

(Ib)

in welcher
$R^1$, $R^2$, $R^3$, $R^4$, $R^5$, $R^6$, $R^7$, $R^8$, A und n die oben angegebene Bedeutung haben,

zu Verbindungen der Formel (Ic)

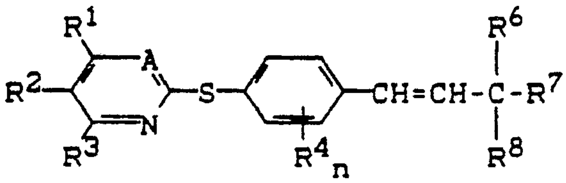

(Ic)

in welcher
$R^1$, $R^2$, $R^3$, $R^4$, $R^5$, $R^6$, $R^7$, $R^8$, A und n die oben angegebene Bedeutung haben,
in Gegenwart eines Halogenierungsmittels, gegebenenfalls in Gegenwart eines Verdünnungsmittels und gegebenenfalls in Gegenwart eines Säurebindemittels umsetzt,
oder daß man

D) Verbindungen der Formel (IV)

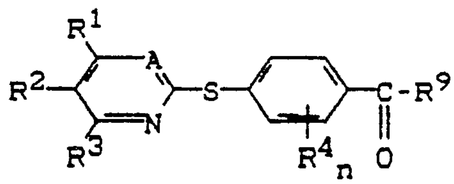

(IV)

in welcher
$R^1$, $R^2$, $R^3$, $R^4$, A und n die oben angegebene Bedeutung haben und
$R^9$ für Wasserstoff oder Alkyl steht,
mit Diolen der Formel (V)

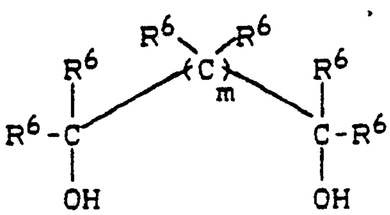

(V)

in welcher
$R^6$ und m die oben angegebene Bedeutung haben,
zu den Verbindungen der Formel (Id)

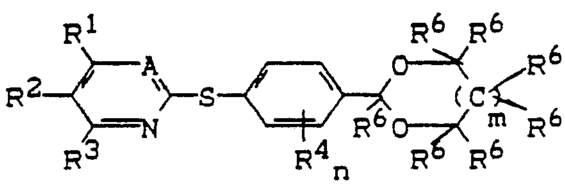

(Id)

in welcher
$R^1$, $R^2$, $R^3$, $R^4$, $R^6$,A m und n die oben angegebene Bedeutung haben,
in Gegenwart eines Verdünnungsmittels und in Gegenwart eines Katalysators umsetzt.

3. Herbizide Mittel, gekennzeichnet durch einen Gehalt an mindestens einem 2-Pyri(mi)dyl-thioarylether der Formel (I) gemäß den Ansprüchen 1 und 2.

4. Verfahren zur Bekämpfung von Unkräutern, dadurch gekennzeichnet, daß man 2-Pyri(mi)dyl-thioarylether der Formel (I) gemäß den Ansprüchen 1 und 2 auf die Unkräuter und/oder ihren Lebensraum einwirken läßt.

5. Verwendung von 2-Pyri(mi)dyl-thioarylethern der Formel (I) gemäß den Ansprüchen 1 und 2 zur Bekämpfung von Unkräutern.

6. Verfahren zur Herstellung von herbiziden Mitteln, dadurch gekennzeichnet, daß man 2-Pyri(mi)dyl-thioarylether der Formel (I) gemäß den Ansprüchen 1 und 2 mit Streckmitteln und/oder oberflächenaktiven Substanzen vermischt.

7. Thiophenole der Formel (IIz)

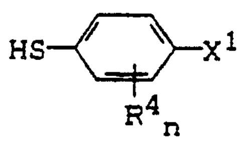

(IIz)

in welcher
$R^4$ für Halogen, Alkyl oder Alkoxy steht, wobei $R^4$ gleich oder verschieden sein kann,
n für eine Zahl 0, 1 oder 2 steht,
$X^1$ für die Gruppe

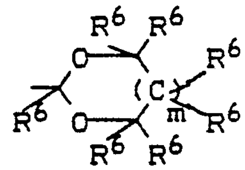

steht, wobei
$R^5$ gleich oder verschieden sein kann und jeweils für Wasserstoff oder Alkyl steht und
m für die Zahl 0 oder 1 steht.

8. Verfahren zur Herstellung von Thiophenolen der Formel (IIz)

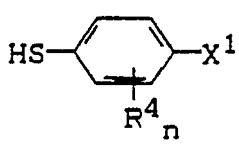

(IIz)

in welcher
$R^4$ für Halogen, Alkyl oder Alkoxy steht, wobei $R^4$ gleich oder verschieden sein kann,
n für eine Zahl 0, 1 oder 2 steht,
$X^1$ für die Gruppe

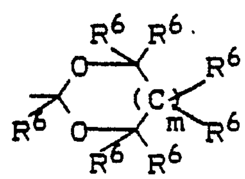

steht, wobei
$R^5$ gleich oder verschieden sein kann und jeweils für Wasserstoff oder Alkyl steht und
m für die Zahl 0 oder 1 steht,
dadurch gekennzeichnet, daß man Verbindungen der Formel (VII)

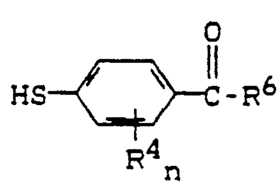

(VII)

in welcher
$R^4$, $R^6$ und n die oben angegebene Bedeutung haben,
mit Diolen der Formel (V)

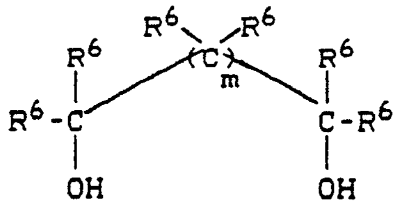
(V)

in welcher
$R^6$ und m die oben angegebene Bedeutung haben,
in Gegenwart eines Lösungsmittels und in Gegenwart einer Säure kondensiert.

9. 4-Carbonyl-[2-pyri(mi)dyl]-thioarylether der Formel (IVa)

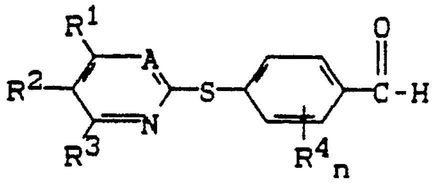
(IVa)

in welcher
$R^1$, $R^2$ und $R^3$ unabhängig voneinander jeweils für Wasserstoff oder Alkyl stehen,
A für eine CH-Gruppe oder ein Stickstoffatom steht,
$R^4$ für Halogen, Alkyl oder Alkoxy steht, wobei $R^4$ gleich oder verschieden sein kann und
n für eine Zahl 0, 1 oder 2 steht.

10. Verfahren zur Herstellung von 4-Carbonyl-[2-pyri(mi)dyl]-thioarylethern der Formal (IVa)

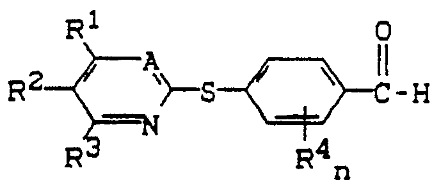
(IVa)

in welcher
$R^1$, $R^2$ und $R^3$ unabhängig voneinander jeweils für Wasserstoff oder Alkyl stehen,
A für eine CH-Gruppe oder ein Stickstoffatom steht,
$R^4$ für Halogen, Alkyl oder Alkoxy steht, wobei $R^4$ gleich oder verschieden sein kann und
n für eine Zahl 0, 1 oder 2 steht,
dadurch gekennzeichnet, daß man 4-[2-Pyri(mi)dylthio]-benzylalkohole der Formel (X)

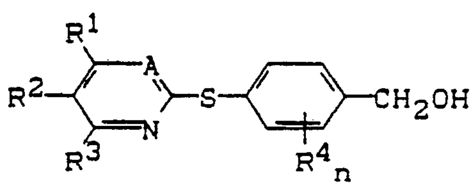
(X)

in welcher
$R^1$, $R^2$, $R^3$, $R^4$, A und n die oben angegebene Bedeutung haben,
in Gegenwart eines Verdünnungsmittels mit Oxidationsmitteln oxidiert.

11. 4-[2-Pyri(mi)dylthio]-benzylalkohole der Formel (X)

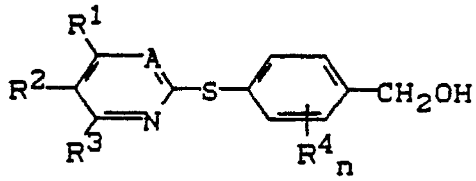

(X)

in welcher
$R^1$, $R^2$ und $R^3$ unabhängig voneinander jeweils für Wasserstoff oder Alkyl stehen,
A für eine CH-Gruppe oder ein Stickstoffatom steht,
$R^4$ für Halogen, Alkyl oder Alkoxy steht, wobei $R^4$ gleich oder verschieden sein kann und
n für eine Zahl 0, 1 oder 2 steht.

12. Verfahren zur Herstellung von 4-[2-Pyri(mi)dylthio]-benzylalkoholen der Formel (X)

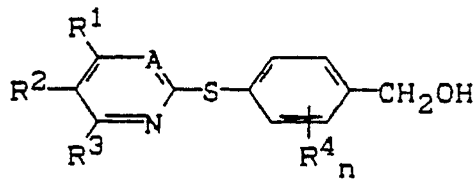

(X)

in welcher
$R^1$, $R^2$ und $R^3$ unabhängig voneinander jeweils für Wasserstoff oder Alkyl stehen,
A für eine CH-Gruppe oder ein Stickstoffatom steht,
$R^4$ für Halogen, Alkyl oder Alkoxy steht, wobei $R^4$ gleich oder verschieden sein kann und
n für eine Zahl 0, 1 oder 2 steht,
dadurch gekennzeichnet, daß man

I) Pyri(mi)din-Derivate der Formel (III)

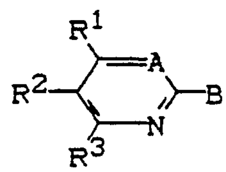

(III)

in welcher
$R^1$, $R^2$, $R^3$, A und B die oben angegebene Bedeutung haben,
mit Thiophenolen der Formel (XI)

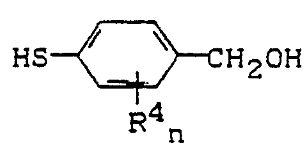

(XI)

in welcher
$R^4$ und n die oben angegebene Bedeutung haben,
in Gegenwart eines Verdünnungsmittels und in Gegenwart eines Säurebindemittels umsetzt
oder daß man

K) 4-[2-pyri(mi)dylthio]-benzoesäureester der Formel (XII)

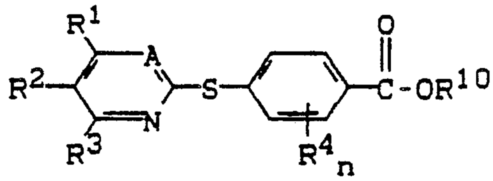

(XII)

in welcher
$R^1$, $R^2$, $R^3$, $R^4$, A und n die oben angegebene Bedeutung haben und
$R^{10}$ für $C_1$-$C_8$-Alkyl oder $C_3$-$C_8$-Cycloalkyl steht,
mit Reduktionsmitteln in Gegenwart eines Verdünnungsmittels umsetzt.